# EUROPEAN PATENT APPLICATION

(11) **EP 4 091 675 A1**
(43) Date of publication of application: **23.11.2022**
(21) Application number: 21186530.8
(22) Date of filing: 19.07.2021
(51) Int. Cl.: A61Q 5/00, A61Q 5/02, A61Q 5/12, A61Q 19/10, A61Q 19/00, A61K 8/33, A61K 8/34, A61K 8/35, A61K 8/49

(54) **COSMETIC COMPOSITION COMPRISING PIROCTONE AND A FRAGRANCE**

(71) Applicant: Clariant International Ltd, 4132 Muttenz (CH)
(72) Inventor: KLUG, Peter, 65926 Frankfurt a. M. (DE); BIRKEL, Susanne, 65926 Frankfurt a. M. (DE)
(74) Representative: Kampen, Daniela

(57) **Abstract**

The present invention relates to a cosmetic composition comprising piroctone and a fragrance as well as to the use of piroctone as a replacement of piroctone olamine in a cosmetic composition.

## Description

The present invention relates to a cosmetic composition comprising piroctone and a fragrance as well as to the use of piroctone as a replacement of piroctone olamine in a cosmetic composition.

Piroctone olamine (Octopirox^{®}) is an effective, nontoxic anti-dandruff agent. It is particularly suitable for the manufacture of anti-dandruff shampoos and hair care products such as hair tonics and cream rinses. Microorganisms such as Malassezia furfur produce enzymes which decompose fats into their respective fatty acids. These and other products of decomposition, such as lipo-peroxides, irritate the scalp. As a result, mitosis and production of keratinocytes increases, leading to desquamation and parakeratosis. Piroctone olamine controls the growth of microorganisms effectively and directly targets the cause of dandruff.

Piroctone olamine is often used in anti-dandruff shampoos as a safe alternative to the commonly used compound zinc pyrithione.

Preservatives are added to most personal care products today in order to inhibit growth of bacteria and fungi. Piroctone olamine has been successfully used for over 30 years because of its good compatibility, antimicrobial functionality, and long shelf life. It is generally considered safe, non-irritating, and non-allergenic.

GB1440975, EP0158481, and WO2006/081969, among others, describe the use of Piroctone Olamine as an anti-dandruff agent and/or as a preservative.

Nevertheless, there is a desire for improved preservation and antifungal systems. In particular, there is a desire for reducing the concentration of such systems - but this requires improved efficacy to ensure that sufficient microbial growth is inhibited. Consumers have generally become more conscious about the content of cosmetic and household products, and they desire reduced levels of 'chemicals', particularly those that may have a reputation for inhibiting the growth of microorganisms.

Octopirox^{®} (piroctone olamine) is compatible with most surfactants, additives and active ingredients used in cosmetics. Compatibility with perfume oils with aldehyde and ketone functionality may be limited in some cases. There is a desire for preservation and antifungal systems that are compatible with perfume oils with aldehyde and ketone functionality.

With the foregoing in mind, there remains a need for more efficacious preservation and antifungal substances, which meet present performance requirements as well as modern consumer desires and expectations.

It has now been found that piroctone is compatible with perfume oils with aldehyde and ketone functionality.

The present invention relates to a cosmetic composition comprising piroctone and a fragrance.

Advantageously, the use of piroctone in a cosmetic composition may allow for a reduction of the amount of fragrance (in particular fragrances comprising an aldehyde or ketone functional group) used in said cosmetic composition. This may lead to cost reductions.

The olamine counter ion in piroctone olamine accounts for approximately 20% of the overall weight of piroctone olamine but does not contribute to its antifungal activity. Piroctone as such does not have an olamine counter ion and therefore the activity to weight ratio is higher than in piroctone olamine.

The cosmetic composition of the present invention comprises piroctone.

Piroctone as used herein is also known as 1-hydroxy-4-methyl-6-(2,4,4-trimethyl)-pentyl-2(1H)-pyridone and refers to a compound of the following formula and can be described with CAS 50650-76-5:

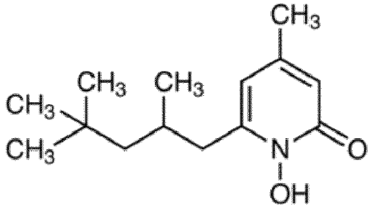

Piroctone can, for example, be prepared as described in WO2019/228988A1.

For example, the cosmetic composition of the invention comprises from 0.01 to 10 wt.-%, preferably from 0.05 to 5 wt.-% of piroctone, based on the total weight of the cosmetic composition.

In preferred embodiments, the cosmetic composition of the invention comprises from 0.05 to 5 wt.-%, preferably from 0.1 to 2 wt.-%, more preferably from 0.1 to 1.0 wt.-%, particularly preferably from 0.1 to 0.5 wt.-% of piroctone, based on the total weight of the cosmetic composition.

The cosmetic composition of the present invention comprises a fragrance.

Preferably, fragrances are fragrant oils. Examples of fragrances are essential oils, flower oils, extracts from plants, fragrances derived from animals, odorants isolated from natural products, semisynthetic odorants (chemically modified odorants) or synthetic odorants (odorants obtained by purely synthetic means). Preferred fragrances are fragrances derived from plants, fragrances derived from animals or semisynthetic odorants. Particularly preferred fragrances are derived from plants.

Fragrances that can be used in the present invention can, for example, be derived from plants. For example, fragrances can be derived from flowers, e.g. lavender, rose, jasmine, neroli; stems and leaves, e.g. geranium, patchouli, petit grain; fruits, e.g. anis, coriander, caroway, juniper; fruit peels, e.g. agrumes, such as bergamot, lemon, orange; seeds, e.g. mace, angelica, celery, cardamom; roots, e.g. angelica, costus, iris, calmus; wood, e.g. sandalwood, guaiac wood, cedar wood, rosewood; herbs and grasses, e.g. tarragon, lemongrass, sage, thyme; needles and branches, e.g. spruce, fir, pine, dwarf-pine; resins and balsams, e.g. galvanum, elemi, benzoin, myrrh, olibanum, opoponax.

Fragrances that can be used in the present invention can, for example, be derived from animals. For example, fragrances can be derived from ambergris, musk, civet, castoreum.

Further examples of fragrances are isoeugenol, vanillin, hydroxycitronellal, citronellol, geranyl acetate, ionones, methylionones.

Further examples of fragrances are citronellal, hydroxy-citronellal, citral, benzaldeyde, vanillin.

In a preferred embodiment, the fragrance comprises an aldehyde or ketone functional group.

Examples of fragrances comprising an aldehyde or ketone functional group are citronellal, hydroxy-citronellal, citral, benzaldeyde, vanillin.

In preferred embodiments, the cosmetic composition of the invention comprises from 0.1 to 5 wt.-%, preferably from 0.1 to 2 wt.-%, more preferably from 0.1 to 1.0 wt.-%, particularly preferably from 0.1 to 0.5 wt.-% of the fragrance, based on the total weight of the cosmetic composition. In another preferred embodiment, the cosmetic composition of the invention comprises from 0.5 to 1.0 wt.-% of the fragrance, based on the total weight of the cosmetic composition.

In one embodiment, the cosmetic composition of the invention is free of piroctone olamine.

In one embodiment, the cosmetic composition of the invention is free of ethanolamine.

In one embodiment, the cosmetic composition of the invention is free of a product of the reaction of an aldehyde or ketone functional group-bearing fragrance with ethanolamine.

For example, the cosmetic composition of the invention may be selected from the group consisting of shampoo, hair conditioner, hair tonic, cream rinse, body wash, bubble bath, bath oil, facial cleanser, cleansing mask, cleansing milk, micellar water, make-up remover, cleansing wipes, perfume, soaps, shaving soaps, shaving foams, cleansing foams, face mask, face cream, hand cream and body lotion. In a particularly preferred embodiment, the cosmetic composition is an anti-dandruff shampoo.

Preferably, the cosmetic composition of the invention is a hair care composition, scalp care composition or skin care composition. More preferably, the cosmetic composition of the invention is a hair care or scalp care composition, particularly preferably a hair care and scalp care composition. Also more preferably, the cosmetic composition of the invention is a skin care composition.

Preferably, the cosmetic composition of the invention is a shampoo composition or hair conditioner composition. More preferably, the cosmetic composition of the invention is a shampoo composition, particularly preferably an anti-dandruff shampoo composition. Also more preferably, the cosmetic composition of the invention is a hair conditioner composition.

In preferred embodiments, the cosmetic composition of the invention is a shampoo composition. The shampoo composition can be in the form of rinse-off products or 'dry shampoo' products, can be opaque or transparent, and can be formulated in a wide variety of product forms, including creams, gels, emulsions, mousses and sprays. Preferably, the shampoo composition is in the form of a rinse-off product. The shampoo composition can, for example, be used on human hair and/or scalp or animal hair, preferably human hair and/or scalp.

In preferred embodiments, the cosmetic composition of the invention is a hair conditioner composition. The hair conditioner composition can be in the form of rinse-off products or leave-on products, can be opaque or transparent, and can be formulated in a wide variety of product forms, including creams, gels, emulsions, mousses and sprays. Preferably, the hair conditioner composition is in the form of a rinse-off product.

In at least one embodiment, the cosmetic composition is in liquid form. In an alternative embodiment, the cosmetic composition is in solid form. Optionally, the cosmetic composition is in powdered or granulated form. This is advantageous in that it is not needed to ship liquid, which is typically heavy, over long distances, which has economic and environmental benefits. A solid form can be achieved by spray drying the composition or by using a rotary evaporator. A solid form can also be achieved by extrusion or pressing. The composition can be converted into liquid form after it has been shipped, e.g. by adding water.

The cosmetic composition of the invention preferably comprises one or more further components (F), which can be in an amount of at least 0.01% by weight, preferably at least 0.05% by weight, more preferably at least 0.1% by weight, even more preferably at least 0.5% by weight of the cosmetic composition.

Preferably, the component (F) is selected from the group consisting of acidity regulators, colorants, conditioning agents, emulsifiers, film formers, glossers, humectants, lubricants, moisturizers, pigments, preservatives, skin penetration enhancers, stabilizers, surfactants, thickeners, and viscosity modifiers. More preferably, the component (F) is selected from the group consisting of acidity regulators, glossers, lubricants, and surfactants.

Suitable lubricants are, for example, fatty alcohol components having 6 to 18 carbon atoms.

The surfactants may, for example, be selected from non-polymeric, cationic quaternary ammonium compounds, in particular cetrimonium chloride (CTAC).

Suitable classical cationic conditioning agents include cationic quaternary ammonium salts. In at least one embodiment, the component (F) is a cationic quaternary ammonium salt. Examples of such quaternary ammonium salts include benzyl triethyl ammonium chloride, cetyl trimethylammonium chloride (cetrimonium chloride, CTAC), behentrimonium chloride (BTAC) or cetylpyridinium chloride.

As cationic components, a variety of cationic polymers are suitable, including quaternized cellulose ethers, copolymers of vinylpyrrolidone, acrylic polymers, including homopolymers or copolymers of dimethyldiallylammonium chloride or acrylamide. Also suitable are various types of homo- or copolymers derived from acrylic or methacrylic acid, acrylamide, methylacrylamide, diacetone-acrylamide.

In at least one embodiment, the component (F) is a glosser. Typical glossers are silicones. Suitable as silicones are volatile or nonvolatile non-ionic silicone fluids, silicone resins, and silicone semisolids or solids. Volatile silicones are linear or cyclic silicones having a measureable vapor pressure, which is defined as a vapor pressure of at least 2 mm of mercury at 20°C. Also suitable are water insoluble nonvolatile silicone fluids including polyalkyl siloxanes, polyaryl siloxanes, polyalkylaryl siloxanes, polyether siloxane copolymers, amine-functional silicones, or mixtures thereof.

The cosmetic composition of the invention may contain from 0.05 to 5%, preferably 0.5 to 5% by weight of at least one oil component. Typical oils are organic oils, which often are esters. The oil component may comprise glyceryl esters of fatty acids, or triglycerides, coconut oil, almond oil, apricot kernel oil, avocado oil, babassu oil, evening primrose oil, camelina sativa seed oil, grape seed oil, macadamia ternifolia seed oil, corn oil, meadowfoam seed oil, mink oil, olive oil, palm kernel oil, safflower oil, sesame oil, soybean oil, sunflower oil, wheat germ oil, and camellia reticulata seed oil.

The cosmetic composition of the invention may contain from 0.05 to 5%, preferably 0.5 to 5% by weight of at least one emulsifier. Preferred emulsifiers are, for example, sorbitan esters.

The cosmetic composition of the invention can contain from 0.1 to 10% by weight, preferably from 0.2 to 5% by weight, more preferably from 0.2 to 3% by weight, also more preferably from 0.5 to 5% by weight of at least one rheology modifying agent, in particular a gelling and thickening agent. Examples are cellulosic thickeners, for example, hydroxyethylcellulose, hydroxypropylcellulose, and carboxymethylcellulose, guar gum, such as hydroxypropylguar, gums of microbial origin, such as xanthan gum and scleroglucan gum, and synthetic thickeners, such as crosslinked homo- or copolymers of acrylic acid and/or of acrylamidopropanesulphonic acid. Other rheology modifying agents include fatty acid amides such as coconut diethanolamide and monoethanolamide, and oxyethylenated monoethanolamide of carboxylic acid alkyl ether.

Rheology modifying agents are also known as structuring materials. Common structuring materials include polymeric materials known as "carbomers", including, for example the cross-linked polyacrylic acid polymers available from Lubrizol Corporation under the trademark Carbopol^{®}. Another class of (meth)acylic acid polymers are alkali-swellable emulsion (ASE) polymers. ASE polymers include, for example, Aculyn^{®} 38 copolymer from Dow. Carbomers and ASE polymers belong to a class of materials known as hydrodynamic thickeners. These hydrodynamic thickeners include acid groups in their polymeric structure that, when deprotonated, form anionic charges that repel each other, causing the polymer chains to expand and entangle. Expansion and chain entanglement can give rise to thickening and suspending effects provided by the deprotonated polymers. The properties of these hydrodynamic thickeners are impacted by their molecular weight, acid group content, degree of cross-linking, and extent of swelling. These thickeners are also known as "space filling" or "volume excluding", and tend to increase both viscosity and yield point as the concentration thereof is increased. In use, hydrodynamic polymers commonly give rise to compositions that exhibit shear thinning or non-Newtonian behavior. Another class of (meth)acrylic acid based rheology modifiers are hydrophobically modified alkali swellable (HASE) polymers. Like ASE polymers, the HASE polymers include acid groups, the deprotonation of which gives rise to polymer swelling. Additionally, the HASE polymers include hydrophobic side groups, chains or blocks that give rise to associative interactions with each other, as well as with other hydrophobic species present in the compositions in which they are employed, for example, hydrophobic groups of surfactants, fatty acids, other thickening agents, and the like. Association creates hydrophobic regions distributed throughout the polymer chain network. This can also help to enhance the properties of the materials as solubilizing agents. Aculyn^{®} 22 and Aculyn^{®} 28 copolymers from Dow and Aqua SF 1^{®} copolymer from Lubrizol Corporation are among the commonly used HASE materials. U.S. Patent 4,529,773 (Witiak et al.) reports alkali-soluble emulsion polymers activated by neutralization to a pH above 6.5, and subsequently acidified in the presence of a surfactant. These are described as useful thickeners in acidic compositions. The polymers are formed from the copolymerization of a monomer system that includes: (1) methacrylic or acrylic acid, (2) methacrylic or acrylic acid ester of a C8-C30 alkyl or, as therein more particularly described, a hydrocarbyl monoether of polyethylene glycol, (3) a C1-C4 alkyl acrylate or methacrylate, and, optionally, (4) a small amount of a polyethylenically unsaturated monomer.

The cosmetic composition of the invention can also comprise as component (F) a fatty compound. The fatty compound may be included in the cosmetic composition at a level of from 0.1 to 20 % by weight, preferably from 1.0 to 10 % by weight. The fatty compound is selected from the group consisting of fatty alcohols (e.g. cetyl alcohol, stearyl alcohol or cetearyl alcohol), fatty acids, fatty alcohol derivatives, fatty acid derivatives, or mixtures thereof.

It is understood that the components disclosed can in some instances fall into more than one classification, e.g., some fatty alcohol derivatives can also be classified as fatty acid derivatives. However, a given classification, is not intended to be a limitation on that particular compound but is done so for convenience of classification and nomenclature. Non-limiting examples are found in International Cosmetic Ingredient Dictionary and Handbook, Fourteenth Edition (2014), and CTFA Cosmetic Ingredient Handbook, Second Edition, 1992. Preferably, fatty alcohols have 14 to 30 or 16 to 22 carbon atoms. These fatty alcohols are saturated and can be linear or branched. Examples of fatty alcohols are cetyl alcohol, stearyl alcohol, behenyl alcohol, and mixtures thereof. Preferred fatty acids have from 10 to 30 or from 12 to 22 carbon atoms. These fatty acids can be saturated and can be linear or branched. Also included herein are salts of these fatty acids. Examples of fatty acids are lauric acid, palmitic acid, stearic acid, behenic acid, sebacic acid, or mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, or mixtures thereof. Examples of fatty alcohol derivatives and fatty acid derivatives include methyl stearyl ether, polyoxyethylene ethers of behenyl alcohol, ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, or mixtures thereof.

The cosmetic composition of the invention may comprise an aqueous carrier. The level and species of the aqueous carrier are selected according to the compatibility with other components and other desired characteristic of the cosmetic composition. The aqueous carrier may, for example, be water or water solutions of lower alkyl alcohols or polyhydric alcohols. The lower alkyl alcohols may, for example, be monohydric alcohols having 1 to 6 carbons, often ethanol and/or isopropanol. The polyhydric alcohols may, for example, be propylene glycol, hexylene glycol, glycerin, and/or propane diol. Preferably, the aqueous carrier is substantially water. Deionized water is preferably used. Water from natural sources, including minerals can also be used, depending on the desired characteristic of the composition. Generally, the cosmetic composition of the invention can comprise up to 80 %, often even up to 95 % by weight of water.

The cosmetic composition of the invention may also include as a further component (F) other components being suitable for rendering the compositions more cosmetically or aesthetically acceptable or to provide them with additional usage benefits. Such other components can generally be used individually at levels of from 0.001 % to 5 % by weight. A wide variety of further components (F) can be formulated into the cosmetic composition of the invention. These include conditioning agents, such as panthenol, panthenyl ethyl ether, proteins, hydrolysed proteins (preferably of vegetable or animal origin, for example hydrolysed collagen or hydrolysed keratin), nutrients; antioxidants, such as vitamin E; emollients, such as PPG-3 myristyl ether, trimethyl pentanol hydroxyethyl ether; hair-fixative polymers, such as amphoteric fixative polymers, cationic fixative polymers, anionic fixative polymers, non-ionic fixative polymers, silicone grafted copolymers; preservatives, such as benzyl alcohol, methyl paraben, propyl paraben, imidazolidinyl urea; pH adjusting agents, such as citric acid, sodium citrate, succinic acid, phosphoric acid, sodium hydroxide, sodium carbonate; salts, in general, such as potassium acetate or sodium chloride; coloring agents; hair oxidizing (bleaching) agents, such as hydrogen peroxide, perborate or persulfate salts; hair reducing agents such as thioglycolates; and sequestering agents, such as disodium ethylene-diamine tetraacetate.

Preferably, salt is present at levels from 0.1 to 1 wt.-% of the total cosmetic composition to adjust the product viscosity. Preferably, NaOH is present at levels from 0.1 to 1 wt.-% of the total cosmetic composition to adjust the pH of the formulation.

The cosmetic composition of the invention may contain as a further component (F) a polysorbate for adjusting rheology, for example, polysorbate-20, polysorbate-21, polysorbate-40, polysorbate-60, or mixtures thereof. The polysorbate can be contained in the cosmetic composition in amounts up to 5% (e.g. 0.1 to 5%) by weight.

The cosmetic composition of the invention can also contain as a further component (F) a polypropylene glycol. Preferred polypropylene glycols are those having a weight average molecular weight of from 200 to 100000 g/mol. The polypropylene glycol may be either water-soluble, water-insoluble, or may have a limited solubility in water, depending upon the degree of polymerization and whether other moieties are attached thereto. The desired solubility of the polypropylene glycol in water will depend in large part upon the form of the composition (e.g., leave-on composition, rinse-off composition). The polypropylene glycol can be included in the cosmetic composition of the invention at a level of up to 10% by weight.

For example, in a rinse-off composition, it is preferred that the polypropylene glycol has a solubility in water at 25°C of less than about 1 g/100 g water, more preferably a solubility in water of less than about 0.5 g/100 g water, and even more preferably a solubility in water of less than about 0.1 g/100 g water. The polypropylene glycol can be included in the cosmetic composition of the invention at a level of up to 10% by weight.

The cosmetic composition of the invention can also contain, as a further component (F), low melting point oil selected from the group consisting of hydrocarbons having from 10 to 40 carbon atoms; unsaturated fatty alcohols having from 10 to 30 carbon atoms such as oleyl alcohol; unsaturated fatty acids having from about 10 to about 30 carbon atoms; fatty acid derivatives; fatty alcohol derivatives; ester oils such as pentaerythritol ester oils, trimethylol ester oils, citrate ester oils, or glyceryl ester oils; poly [alpha]-olefin oils; and mixtures thereof. Preferred low melting point oils are selected from the group consisting of ester oils such as pentaerythritol ester oils, trimethylol ester oils, citrate ester oils, or glyceryl ester oils; poly [alpha]-olefin oils; and mixtures thereof. Particularly useful pentaerythritol ester oils and trimethylol ester oils are pentaerythritol tetraisostearate, pentaerythritol tetraoleate, trimethylolpropane triisostearate, trimethylolpropane trioleate, or mixtures thereof. Particularly useful glyceryl esters are triisostearin, triolein or trilinolein.

The cosmetic composition of the invention can also contain, as a further component (F), a cationic polymer. Cationic polymers may be present in the cosmetic composition of the invention for further enhancing deposition performance.

Suitable cationic polymers may be homopolymers which are cationically substituted or may be formed from two or more types of monomers. The weight average (Mw) molecular weight of the polymers will generally be between 100 000 and 2 million g/mol. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain non-cationic spacer monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol. The cationic amines can be primary, secondary or tertiary amines, depending upon the particular species and the pH of the composition. In general, secondary and tertiary amines, especially tertiary, are preferred. Amine substituted vinyl monomers and amines can be polymerized in the amine form and then converted to ammonium by quaternization. The cationic polymers can comprise mixtures of monomer units derived from amine- and/or quaternary ammonium-substituted monomer and/or compatible spacer monomers.

Suitable cationic polymers include, for example cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; mineral acid salts of amino-alkyl esters of homo- and co-polymers of unsaturated carboxylic acids having from 3 to 5 carbon atoms, (as described in US4009256A1 from NAT STARCH CHEM CORP); cationic polyacrylamides (as described in WO95/22311A1 Unilever PLC).

Other cationic polymers that can be used include cationic polysaccharide polymers, such as cationic cellulose derivatives, cationic starch derivatives, and cationic guar gum derivatives.

Cationic polysaccharide polymers suitable for use in the cosmetic composition of the invention include monomers of the formula: A-O-[R-N⁺(R1)(R2)(R3)X⁻], wherein: A is an anhydroglucose residual group, such as a starch or cellulose anhydroglucose residual. R is an alkylene, oxyalkylene, polyoxyalkylene, or hydroxyalkylene group, or combination thereof. R1, R2 and R3 independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R1, R2 and R3) is preferably about 20 or less, and X⁻ is an anionic counterion. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the trade name Polymer LM-200. Other suitable cationic polysaccharide polymers include quaternary nitrogen-containing cellulose ethers (e.g. as described in US3962418 from L'Oréal), and copolymers of etherified cellulose and starch (e.g. as described in US3958581 from L'Oréal).

A particularly suitable type of cationic polysaccharide polymer that can be used is a cationic guar gum derivative, such as guar hydroxypropyltrimethylammonium chloride (commercially available from Solvay in their JAGUAR trade named series). Examples of such materials are JAGUAR C13S, JAGUAR C14, JAGUAR C15, JAGUAR C17, JAGUAR C16, JAGUAR CHT and JAGUAR C162.

Mixtures of any of the above cationic polymers may be used. Cationic polymer may be present in the cosmetic composition of the invention at levels of from 0.01 to 5 wt.-%, preferably from 0.05 to 1 wt.-%, more preferably from 0.08 to 0.5 wt.-% by total weight of cationic polymer based on the total weight of the cosmetic composition.

In at least one embodiment, the cationic polymers have a number average molecular weight of at least about 5000 g/mol, typically from 10000 g/mol to 10 million g/mol and are selected from the group consisting of copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol, maleic anhydride, propylene glycol, and ethylene glycol. Preferred cationic polymers are cationic celluloses, cationic starches, and cationic guar gums. Commercially available cationic guar polymers are e.g. Jaguar^{®} from Solvay.

In at least one embodiment, the cosmetic composition of the invention comprises a surfactant system. In at least one embodiment, the surfactant system comprises a surfactant selected from the group consisting of anionic surfactants, cationic surfactants, non-ionic surfactants, zwitterionic surfactants and/or amphoteric surfactants. In at least one embodiment, the cosmetic composition of the invention comprises a total amount of surfactant of from 0.01 wt.-% to 70 wt.-%, from 0.1 wt.-% to 40%, from 1 wt.-% to 30%, from 2 wt.-% to 20 wt.-%.

In at least one embodiment, the cosmetic composition of the invention comprises an anionic surfactant. In at least one embodiment, the anionic surfactant is selected from the group consisting of (C10-C20)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, alpha-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, acylglutamates, and mixtures thereof. The anionic surfactants (and their mixtures) can be used in the form of their water-soluble or water-dispersible salts, examples being the sodium, potassium, magnesium, ammonium, mono-, di-, and triethanolammonium, and analogous alkylammonium salts. In at least one embodiment, the anionic surfactant is the salt of an anionic surfactant comprising 12 to 14 carbon atoms. In at least one embodiment, the anionic surfactant is selected from the group consisting of sodium lauryl sulfate, sodium laureth sulfate, sodium tridecyl sulfate, sodium trideceth sulfate, sodium myristyl sulfate, sodium myreth sulfate, and mixtures thereof. Typical anionic surfactants for use in the cosmetic composition of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecyl benzene sulphonate, triethanolamine dodecylbenzene sulphonate, sodium cocoyl isethionate, sodium lauryl isethionate, lauryl ether carboxylic acid and sodium N-lauryl sarcosinate. Preferred anionic surfactants are selected from sodium lauryl sulphate and sodium lauryl ether sulphate(n)EO, where n is from 1 to 3; more preferably sodium lauryl ether sulphate(n)EO, where n is from 1 to 3; most preferably sodium lauryl ether sulphate(n)EO, where n=1. Preferably, the level of alkyl ether sulphate is from 0.5 wt.-% to 25 wt.-% of the total composition, more preferably from 3 wt.-% to 18 wt.-%, most preferably from 6 wt.-% to 15 wt.-% of the total composition.

The total amount of anionic surfactant in the cosmetic composition of the invention may range from 0.5 wt.-% to 45 wt.-%, more preferably from 1.5 wt.-% to 20 wt.-%.

The cosmetic composition of the invention may comprise fatty acyl isethionate, if present, preferably at a level of from 1 to 10 wt.-%, more preferably from 2 to 8 wt.-%, most preferably from 2.5 to 7.5 wt.-%. A preferred fatty acyl isethionate product comprises fatty acyl isethionate surfactant at a level of from 40 to 80 wt.-% of the product, as well as free fatty acid and/or fatty acid salt at a level of from 15 to 50 wt.-%. Preferably, greater than 20 wt.-% and less than 45 wt.-%, more preferably greater than 25 wt.-% and less than 45 wt.-% of the fatty acyl isethionate are of chain length greater than or equal to C16; and greater than 50 wt.-%, preferably greater than 60 wt.-% of the free fatty acid/soap is of chain length C16 to C20. In addition, the product may contain isethionate salts, which are present typically at levels less than 5 wt.-%, and traces (less than 2 wt.-%) of other impurities.

Preferably, a mixture of aliphatic fatty acids is used for the preparation of commercial fatty acyl isethionate surfactants. The resulting fatty acyl isethionate surfactants (e.g., resulting from the reaction of alkali metal isethionate and aliphatic fatty acid) preferably should have more than 20 wt.-%, preferably more than 25 wt.-%, but no more than 45 wt.-%, preferably 35 wt.-% (on basis of fatty acyl isethionate reaction product) of fatty acyl group with 16 or greater carbon atoms to provide both excellent lather and mildness of the resulting fatty acyl isethionate product. These longer chain fatty acyl isethionate surfactants and fatty acids, i.e. fatty acyl group and fatty acid with 16 or more carbons, can typically form insoluble surfactant/fatty acid crystals in water at ambient temperatures.

In at least one embodiment, the cosmetic composition of the invention comprises an acylglycinate surfactant. In at least one embodiment, the acylglycinate surfactant conforms to the formula (Y): 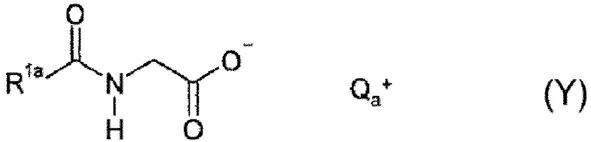 wherein
R^{1a} is a linear or branched, saturated alkyl group having 6 to 30, preferably 8 to 22, particularly preferably 8 to 18 carbon atoms or is a linear or branched, mono- or polyunsaturated alkenyl group having 6 to 30, preferably 8 to 22, particularly preferably 12 to 18 carbon atoms, and
Qₐ⁺ is a cation.

In at least one embodiment, Qₐ⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. In at least one embodiment, the acylglycinate surfactant is selected from sodium cocoylglycinate and potassium cocoylglycinate. In at least one embodiment, the acylglycinate surfactant is selected from those conforming to formula (Y), wherein R is C12 alkyl or C14 alkyl. In at least one embodiment, the acylglycinate surfactant is selected from those conforming to formula (Y), wherein R is C16 alkyl or C18 alkyl.

In at least one embodiment, the cosmetic composition comprises from 0.01 wt.-% to 30 wt.-%, or from 1 wt.-% to 25 wt.-%, preferably from 5 wt.-% to 20 wt.-%, more preferably from 12 wt.-% to 18 wt.-% anionic surfactant.

In at least one embodiment, the cosmetic composition of the invention comprises a glutamate surfactant corresponding to formula (Z) or a salt thereof: 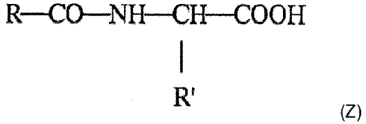 wherein R' is HOOC-CH2-CH2- or M⁺⁻OOC-CH2-CH2- wherein M⁺ is a cation; and wherein R is a linear or branched, saturated alkyl group having 6 to 30, preferably 8 to 22, more preferably 8 to 18 carbon atoms or is a linear or branched, mono- or polyunsaturated alkenyl group having 6 to 30, preferably 8 to 22, more preferably 12 to 18 carbon atoms. In at least one embodiment, M⁺ is a metal cation. In at least one embodiment, M⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. In at least one embodiment, the glutamate surfactant is selected from sodium cocoyl glutamate and potassium cocoyl glutamate. In at least one embodiment, the glutamate surfactant is selected from those conforming to formula (Z), wherein R is C12 alkyl or C14 alkyl. In at least one embodiment, the glutamate surfactant is selected from those conforming to formula (Z), wherein R is C16 alkyl or C18 alkyl.

In at least one embodiment, the cosmetic composition of the invention comprises a non-ionic surfactant. Non-ionic surfactants may be present in the range 0 to 5 wt.-%. Non-ionic surfactants that can be included in the cosmetic composition of the invention include condensation products of aliphatic primary or secondary linear or branched chain alcohols or phenols with alkylene oxides, usually ethylene oxide and generally having from 6 to 30 ethylene oxide groups. Alkyl ethoxylates are particularly preferred. Most preferred are alky ethoxylates having the formula

R-(OCH2CH2)nOH,

where R is an alkyl chain of C12 to C15, and n is 5 to 9. Other suitable non-ionic surfactants include mono- or di-alkyl alkanolamides. Examples include coco mono- or diethanolamide and coco mono-isopropanolamide.

Further non-ionic surfactants which can be included in the cosmetic composition of the invention are alkyl polyglycosides (APGs). Typically, APG is one which comprises an alkyl group connected (optionally via a bridging group) to a block of one or more glycosyl groups. Preferred APGs are defined by the following formula:

RO-(G)n

wherein R is a branched or straight chain alkyl group which may be saturated or unsaturated, and G is a saccharide group. R may represent a mean alkyl chain length of from about C5 to about C20. Preferably R represents a mean alkyl chain length of from about C9 to about C12. G may be selected from C5 or C6 monosaccharide residues, and is preferably a glucoside. G may be selected from the group comprising glucose, xylose, lactose, fructose, mannose and derivatives thereof. Preferably G is glucose. The degree of polymerisation, n, may have a value of from about 1 to about 10 or more. Most preferably the value of n lies between about 1.3 to about 1.5. Suitable alkyl polyglycosides for use in the invention are commercially available and include for example those materials identified as: Oramix NS10 ex Seppic; Plantaren 1200 and Plantaren 2000 ex BASF.

Other sugar-derived non-ionic surfactants which can be included in the cosmetic composition of the invention include fatty (e.g. C10-C18) N-alkyl (C1-C6) polyhydroxy fatty acid amides, such as C12-C18 N-methyl glucamides, as described for example in WO9206154 and US5194639, and N-alkoxy polyhydroxy fatty acid amides.

In at least one embodiment, the non-ionic surfactant has an HLB (Hydrophilic Lipophilic Balance) of greater than 12. Optionally, the non-ionic surfactant is selected from the group consisting of ethoxylated or ethoxylated/propoxylated fatty alcohols with a fatty chain comprising from 12 to 22 carbon atoms, ethoxylated sterols, such as stearyl- or lauryl alcohol (EO-7), PEG-16 soya sterol or PEG-10 soya sterol, polyoxyethylene polyoxypropylene block polymers (poloxamers), and mixtures thereof.

In at least one embodiment, the non-ionic surfactant is selected from the group consisting of ethoxylated fatty alcohols, fatty acids, fatty acid glycerides or alkylphenols, in particular addition products of 2 to 30 mol of ethylene oxide and/or 1 to 5 mol of propylene oxide onto C8- to C22-fatty alcohols, onto C12- to C22-fatty acids or onto alkyl phenols having 8 to 15 carbon atoms in the alkyl group, C12- to C22-fatty acid mono- and diesters of addition products of 1 to 30 mol of ethylene oxide onto glycerol, addition products of 5 to 60 mol of ethylene oxide onto castor oil or onto hydrogenated castor oil, fatty acid sugar esters, in particular esters of sucrose and one or two C8- to C22-fatty acids, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate, esters of sorbitan and one, two or three C8- to C22-fatty acids and a degree of ethoxylation of from 4 to 20, polyglyceryl fatty acid esters, in particular of one, two or more C8- to C22-fatty acids and polyglycerol having preferably 2 to 20 glyceryl units, alkyl glucosides, alkyl oligoglucosides and alkyl polyglucosides having C8 to C22-alkyl groups, e.g. decylglucoside or laurylglucoside, and mixtures thereof.

In at least one embodiment, the non-ionic surfactant is selected from the group consisting of fatty alcohol ethoxylates (alkylpolyethylene glycols), alkylphenol polyethylene glycols, alkylmercaptan polyethylene glycols, fatty amine ethoxylates (alkylaminopolyethylene glycols), fatty acid ethoxylates (acylpolyethylene glycols), polypropylene glycol ethoxylates (Pluronics^{®}), fatty acid alkylol amides, (fatty acid amide polyethylene glycols), N-alkyl-, N-alkoxypoly-hydroxy-fatty acid amide, sucrose esters, sorbitol esters, polyglycol ethers, and mixtures thereof.

In at least one embodiment, the cosmetic composition of the invention comprises a fatty N-methyl-N-glucamide surfactant. In at least one embodiment, the fatty N-methyl-N-glucamide surfactant conforms to the formula (X): 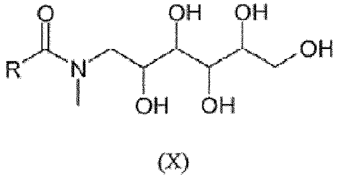 wherein
R is a linear or branched alkyl or alkenyl group having from 3 to 30 carbon atoms. In at least one embodiment, R is an alkyl group having from 3 to 30 carbon atoms. In at least one embodiment, R is a saturated aliphatic hydrocarbon group which can be linear or branched and can have from 3 to 20 carbon atoms in the hydrocarbon chain, preferably linear or branched. Branched means that a lower alkyl group such as methyl, ethyl or propyl is present as substituent on a linear alkyl chain. In at least one embodiment, R is selected from the group consisting of 1-propyl, 2-propyl, 1 butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2 methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3 methyl-2-pentyl, 4-methyl-2-pentyl, 2 methyl-3-pentyl, 3-methyl-3-pentyl, 2,2 dimethyl-1-butyl, 2,3-dimethyl-1-butyl, 3,3 dimethyl-1-butyl, 2-ethyl-1-butyl, 2,3-dimethyl-2-butyl, 3,3-dimethyl-2-butyl, 1 heptyl, 1-octyl, 1-nonyl, 1-decyl, 1 undecyl, 1-dodecyl, 1-tetradecyl, 1-hexadecyl and 1-octadecyl. Suitable fatty N methyl-N-glucamide surfactants are described in WO2013/178700 and EP 0 550 637, which are incorporated herein by reference. In at least one embodiment, the N-methyl-N-glucamide surfactant is selected from those conforming to formula (X), wherein R is C12 alkyl or C14 alkyl. In at least one embodiment, the N-methyl-N-glucamide surfactant is selected from those conforming to formula (X), wherein R is C16 alkyl or C18 alkyl.

In at least one embodiment, the cosmetic composition of the invention comprises from 1 wt.-% to 20 wt.-%, more preferably from 2 wt.-% to 10 wt.-%, even more preferably from 3 wt.-% to 7 wt.-% non-ionic surfactant.

Amphoteric or zwitterionic surfactant can be included in the cosmetic composition of the invention in an amount ranging from 0.5 wt.-% to about 8 wt.-%, preferably from 1 wt.-% to 4 wt.-% of the total composition.

In at least one embodiment, the amphoteric surfactants are selected from the group consisting of N-(C₁₂-C₁₈)-alkyl-beta-aminopropionates and N-(C₁₂-C₁₈)-alkyl-beta-iminodipropionates as alkali metal salts and mono-, di-, and trialkylammonium salts; N-acylaminoalkyl-N,N-dimethylacetobetaine, preferably N-(C₈-C₁₈)-acylaminopropyl-N,N-dimethylacetobetaine; (C₁₂-C₁₈)-alkyl-dimethyl-sulfopropylbetaine; amphosurfactants based on imidazoline (trade name: Miranol^{®}, Steinapon^{®}), preferably the sodium salt of 1-(beta-carboxymethyloxyethyl)-1-(carboxymethyl)-2-laurylimidazolinium; amine oxide, e.g., (C₁₂-C₁₈)-alkyl-dimethylamine oxide, fatty acid amidoalkyldimethylamine oxide, and mixtures thereof.

In at least one embodiment, the cosmetic composition of the invention comprises a betaine surfactant. Optionally, the betaine surfactant is selected from C8- to C18-alkylbetaines. In at least one embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)alphacarboxyethylbetaine and combinations thereof. Optionally, the betaine surfactant is selected from C8- to C18-sulfobetaines. In at least one embodiment, the betaine surfactant is selected from the group consisting of cocodimethylsulfopropylbetaine, stearyldimethylsulfopropylbetaine, lauryldimethylsulfoethylbetaine, laurylbis(2-hydroxyethyl)sulfopropylbetaine, and combinations thereof. Optionally, the betaine surfactant is selected from carboxyl derivatives of imidazole, the C8- to C18-alkyldimethylammonium acetates, the C8- to C18 alkyldimethylcarbonylmethylammonium salts, and the C8- to C18-fatty acid alkylamidobetaines, and mixtures thereof. Optionally, the C8- to C18-fatty acid alkylamidobetaine is selected from coconut fatty acid amidopropylbetaine, N-coconut fatty acid amidoethyl-N-[2-(carboxymethoxy)ethyl]glycerol (CTFA name: cocoamphocarboxyglycinate), and mixtures thereof. A particularly preferred amphoteric or zwitterionic surfactant is cocamidopropyl betaine. Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

In at least one embodiment, the cosmetic composition of the invention comprises from 0.5 wt.-% to 20 wt.-%, preferably from 1 wt.-% to 10 wt.-% amphoteric surfactant.

In at least one embodiment, the cosmetic composition of the invention comprises a surfactant system. In at least one embodiment, the surfactant system comprises at least one surfactant selected from the group consisting of lauryl sulfate, laureth sulfate, cocamidopropyl betaine, sodium cocoylglutamate, lauroamphoacetate, and mixtures thereof. In at least one embodiment, the surfactant system comprises sodium laureth sulfate, sodium lauryl sulfate, and optionally cocamidopropyl betaine. In at least one embodiment, the surfactant system comprises sodium laureth sulfate, potassium cocoylglutamate, and cocamidopropyl betaine.

In at least one embodiment, the cosmetic composition of the invention contains as a further component a silicone compound. The cosmetic composition can comprise up to 5% (e.g. 0.1 to 5%) by weight of a silicone compound. Suitable silicone compounds include polyalkyl or polyaryl siloxanes. The preferred silicone compounds are polydimethylsiloxane, polydiethylsiloxane, and polymethylphenylsiloxane, e.g. available from Wacker (Germany) or Dow Corning, such as Xiameter PMX DC 200. Silicone compounds can be available as silicone oils or emulsions. The silicone compounds may further be incorporated in the present composition in the form of an emulsion, wherein the emulsion is pre-made and added to the formulation, or made during the formulation process by mechanical mixing with or without the aid of an additional surfactant selected from anionic surfactants, non-ionic surfactants, cationic surfactants, and mixtures thereof.

In at least one embodiment, the cosmetic composition of the invention contains silicone conditioning agents. Preferably, these are emulsified droplets of a silicone conditioning agent. These are for enhancing conditioning performance.

Suitable silicones include polydiorganosiloxanes, in particular polydimethylsiloxanes, which have the CTFA designation dimethicone. Also suitable for use in the cosmetic composition of the invention are polydimethyl siloxanes having hydroxyl end groups, which have the CTFA designation dimethiconol. Also suitable for use in the cosmetic composition of the invention are silicone gums having a slight degree of cross-linking, as are described for example in WO 96/31188. The viscosity of the emulsified silicone itself (not the emulsion or the final composition) is typically at least 10,000 cSt at 25°C. The viscosity of the silicone itself is preferably at least 60,000 cSt, most preferably at least 500,000 cSt, ideally at least 1,000,000 cSt. Preferably, the viscosity does not exceed 1×10⁹ cSt for ease of formulation. Emulsified silicones for use in the cosmetic composition of the invention will typically have an average silicone droplet size in the composition of less than 30, preferably less than 20, more preferably less than 10 micron, ideally from 0.01 to 1 micron. Silicone emulsions having an average silicone droplet size of less than 0.15 micron are generally termed microemulsions.

Silicone particle size may be measured by means of a laser light scattering technique, for example using a 2600D Particle Sizer from Malvern Instruments. Examples of suitable pre-formed emulsions include Xiameter MEM 1785 and microemulsion DC2-1865 available from Dow Corning. These are emulsions / microemulsions of dimethiconol. Cross-linked silicone gums are also available in a pre-emulsified form, which is advantageous for ease of formulation. A further preferred class of silicones for inclusion in the cosmetic composition of the invention are amino functional silicones. By "amino functional silicone" is meant a silicone containing at least one primary, secondary or tertiary amine group, or a quaternary ammonium group. Examples of suitable amino functional silicones include polysiloxanes having the CTFA designation "amodimethicone". Specific examples of amino functional silicones suitable for use in the cosmetic composition of the invention are the aminosilicone oils DC2-8220, DC2-8166 and DC2-8566 (all ex Dow Corning). Suitable quaternary silicone polymers are described in EP-A-0 530 974. A preferred quaternary silicone polymer is K3474, ex Goldschmidt.

Also suitable are emulsions of amino functional silicone oils with non-ionic and/or cationic surfactants. Pre-formed emulsions of amino functional silicones are also available from suppliers of silicone oils such as Dow Corning and General Electric. Specific examples include DC939 Cationic Emulsion and the non-ionic emulsions DC2-7224, DC2- 8467, DC2-8177 and DC2-8154 (all ex Dow Corning).

Combination of amino and non-amino functional silicones may also be used.

The total amount of silicone is preferably from 0.01 wt.-% to 10 wt.-% of the total composition, more preferably from 0.1 wt.-% to 5 wt.-%, most preferably from 0.5 wt.-% to 3 wt.-%.

In at least one embodiment, the cosmetic composition of the invention comprises a preservative or preservative system. Examples of suitable preservatives include benzyl alcohol, phenoxyethanol, parabens, benzoic acid/sodium benzoate, sorbic acid/potassium sorbate, and other organic acids used to provide antimicrobial protection. In at least one embodiment, the cosmetic composition comprises from 0.01 to 5 wt.-%, particularly preferably from 0.05 to 1 wt.-% of at least one preservative. Suitable preservatives are the substances listed in the International Cosmetic Ingredient Dictionary and Handbook, 9th Edition with the function "preservatives". In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the cosmetic composition comprises a preservative selected from the group consisting of cetyltrimethyl ammonium chloride, cetylpyridinium chloride, benzethonium chloride, diisobutylethoxyethyldimethyl benzylammonium chloride, sodium N-lauryl sarcosinate, sodium-N-palmethylsarcosinate, lauroylsarcosine, N-myristoylglycine, potassium-N-laurylsarcosine, trimethylammonium chloride, sodium aluminium chlorohydroxylactate, triethylcitrate, tricetylmethylammonium chloride, 2,4,4'-trichloro-2'-hydroxydiphenylether (Triclosan), phenoxyethanol, 3,4,4'-trichlorocarbanilide (Triclocarban), diaminoalkylamide, L-lysine hexadecylamide, heavy metal citrate salts, zinc salts, zinc phenol sulfate, farnesol, naftifine, terbinafine, selenium disulfide, methylchloroisothiazolinone, methylisothiazolinone, methyldibromo glutaronitrile, AgCI, chloroxylenol, sodium salts of diethylhexylsulfosuccinate, sodiumbenzoate, phenoxyethanol, benzyl alcohol, phenoxyisopropanol, paraben, such as butyl-, ethyl-, methyl- und propylparaben, and their salts, sorbic acid, benzoic acid, lactic acid, imidazolidinyl urea, diazolidinyl urea, dimethylol dimethyl hydantoin (DMDMH), sodium salts of hydroxymethyl glycinate, hydroxyethylglycine of sorbic acid and combinations thereof. In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the cosmetic composition of the invention is substantially free of parabens.

The cosmetic composition of the invention may also comprise a dispersed, non-volatile, water-insoluble oily conditioning agent. By "insoluble" is meant that the material is not soluble in water (distilled or equivalent) at a concentration of 0.1 % (w/w), at 25°C.

Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C2-C6 alkenyl monomers. Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols. Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used. Particularly preferred fatty esters are mono-, di- and triglycerides, more specifically the mono-, di-, and tri-esters of glycerol and long chain carboxylic acids such as C8-C22 carboxylic acids. Preferred materials include cocoa butter, palm stearin, sunflower oil, soybean oil and coconut oil.

The oily or fatty material may be present at a level of from 0.05 to 10 wt.-%, preferably from 0.2 to 5 wt.-%, more preferably from 0.5 to 3 wt.-%, based on the total weight of the cosmetic composition.

In a particularly preferred embodiment, the composition of the invention is a shampoo composition.

In at least one embodiment, the shampoo composition comprises from 1 to 99%, preferably from 5 to 95%, more preferably from 10 to 90% by weight of the total composition of water, and from 0.1 to 99%, preferably from 1 to 95%, more preferably from 5 to 90%, often from 5 to 25% by weight of the total composition of a cleansing surfactant. Suitable cleansing surfactants are generally anionic, amphoteric, betaine, or zwitterionic surfactants. For example, the anionic surfactants are alkyl ether or alkyl ether sulfates, such as sodium lauryl sulfate, or other compounds described above.

In at least one embodiment, the shampoo composition comprises one or more further cosmetically acceptable components (F), which can be present in an amount of at least 0.5% by weight, or from 0.5 to 20% by weight, by total weight of the shampoo composition. Preferably, the component (F) is selected from the group consisting of cleansing ingredients, acidity regulators, colorants, conditioning agents, emulsifiers, film formers, glossers, humectants, lubricants, moisturizers, pigments, preservatives, hair penetration enhancers, scalp actives, stabilizers, surfactants, thickeners, viscosity modifiers, and combinations thereof. More preferably, the component (F) is selected from the group consisting of surfactants, viscosity-modifying polymers and conditioning ingredients. In at least one embodiment, the shampoo composition comprises from 0.05 to 5 wt.-%, preferably from 0.1 to 2 wt.-%, more preferably from 0.1 to 1.0 wt.-%, particularly preferably from 0.1 to 0.5 wt.-% of piroctone, from 0.1 to 5 wt.-%, preferably from 0.1 to 2 wt.-%, more preferably from 0.1 to 1.0 wt.-%, particularly preferably from 0.1 to 0.5 wt.-% of the fragrance, and at least 0.5% by weight of one or more further components (F) selected from the group consisting of surfactants, polymers, conditioning agents, actives, acidity regulators, lubricants, moisturizers, oils, preservatives, sequestrants, strengtheners, sun protectors, and combinations thereof.

In at least one embodiment, the shampoo composition comprises further cosmetically acceptable components (F) being cleansing ingredients. In at least one embodiment, the shampoo composition comprises from 0.05 to 20% by weight of cleansing ingredients, based on the total weight of the shampoo composition. In at least one embodiment of the shampoo composition, the level of cleansing ingredient is from 1% to 20% by weight, preferably from 5% to 18%, more preferably from 8% to 16%, based on the total weight of the shampoo composition. In at least one embodiment, the cleansing ingredient is selected from the group consisting of non-polymeric surfactants, saponins, polymeric surfactants, and combinations thereof. Preferably, the cleansing ingredient comprises or consists of surfactants.

In at least one embodiment, the shampoo composition comprises from 0.05 to 5 wt.-%, preferably from 0.1 to 2 wt.-%, more preferably from 0.1 to 1.0 wt.-%, particularly preferably from 0.1 to 0.5 wt.-% of piroctone, from 0.1 to 5 wt.-%, preferably from 0.1 to 2 wt.-%, more preferably from 0.1 to 1.0 wt.-%, particularly preferably from 0.1 to 0.5 wt.-% of the fragrance, and at least 0.5 % by weight of surfactants, preferably cleansing anionic or non-ionic surfactants, such as sodium laureth sulphate, sodium lauryl sulphate, ammonium laureth sulphate, ammonium lauryl sulphate, olefin sulfonates, olefin sulfates, laureth-3 or 4, cocamide DEA, glucosides, cocamidopropyl betaine, coco betaine, cocoamphodipropionate, sodium methyl 2-sulfolaurate and other laurates, sulfoacetates, sulfosuccinates, lactylates, sultaines, caprylates/ caprates, isethionates, glutamates, taurates, sarcosinates, glucamides, and combinations thereof.

In at least one embodiment, the shampoo composition is silicone-free. In at least one embodiment, the shampoo composition is sulfate-free. In at least one embodiment, the shampoo composition is silicone-free and sulfate-free.

In at least one embodiment, the shampoo composition comprises, based on the total weight of the shampoo composition:
(i) from 0.05 to 5 wt.-%, preferably from 0.1 to 2 wt.-%, more preferably from 0.1 to 1.0 wt.-%, particularly preferably from 0.1 to 0.5 wt.-% of piroctone;
(ii) from 0.1 to 5 wt.-%, preferably from 0.1 to 2 wt.-%, more preferably from 0.1 to 1.0 wt.-%, particularly preferably from 0.1 to 0.5 wt.-% of the fragrance;
(iii) from 5 wt.-% to 20 wt.-% of one or more anionic surfactants;
(iv) at least 50 wt.-% water; and
(v) at least one further cosmetically acceptable component (F) selected from the group consisting of silicone, cationic polymer, rheology modifying agent, and an amphoteric or zwitterionic surfactant.

In at least one embodiment, the shampoo composition comprises, based on the total weight of the shampoo composition:
(i) from 0.05 to 5 wt.-%, preferably from 0.1 to 2 wt.-%, more preferably from 0.1 to 1.0 wt.-%, particularly preferably from 0.1 to 0.5 wt.-% of piroctone;
(ii) from 0.1 to 5 wt.-%, preferably from 0.1 to 2 wt.-%, more preferably from 0.1 to 1.0 wt.-%, particularly preferably from 0.1 to 0.5 wt.-% of the fragrance;
(iii) from 5 wt.-% to 20 wt.-% of one or more anionic surfactants;
(iv) at least 50 wt.-% water;
(v) at least one further component selected from the group consisting of silicone, cationic polymer, rheology modifying agent, and an amphoteric or zwitterionic surfactant; and
(vi) at least one further cosmetically acceptable component (F).

In at least one embodiment, the shampoo composition consists of, based on the total weight of the shampoo composition:
(i) from 0.05 to 5 wt.-%, preferably from 0.1 to 2 wt.-%, more preferably from 0.1 to 1.0 wt.-%, particularly preferably from 0.1 to 0.5 wt.-% of piroctone;
(ii) from 0.1 to 5 wt.-%, preferably from 0.1 to 2 wt.-%, more preferably from 0.1 to 1.0 wt.-%, particularly preferably from 0.1 to 0.5 wt.-% of the fragrance;
(iii) from 5 wt.-% to 20 wt.-% of one or more anionic surfactants;
(iv) at least 50 wt.-% water;
(v) at least one further component selected from the group consisting of silicone, cationic polymer, rheology modifying agent, and an amphoteric or zwitterionic surfactant; and
(vi) at least one further cosmetically acceptable component (F) selected from the group consisting of conditioning agents, such as panthenol, panthenyl ethyl ether, proteins, hydrolysed proteins (preferably of vegetable or animal origin, for example hydrolysed collagen or hydrolysed keratin), nutrients; antioxidants, such as vitamin E; emollients, such as PPG-3 myristyl ether, trimethyl pentanol hydroxyethyl ether; hair-fixative polymers such as amphoteric fixative polymers, cationic fixative polymers, anionic fixative polymers, non-ionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents; hair oxidizing (bleaching) agents, such as hydrogen peroxide, perborate and persulfate salts; hair reducing agents, such as the thioglycolates; and sequestering agents, such as disodium ethylene-diamine tetraacetate.

The cosmetic composition of the invention can be manufactured by methods known in the art. For example, the cosmetic composition of the invention can be manufactured by mixing its ingredients. For example, piroctone and the fragrance can be mixed with the other ingredients of the cosmetic composition of the invention.

Piroctone, which is comprised in the cosmetic composition of the invention, is preferably used as an anti-dandruff agent and/or as a preservative. In one embodiment, piroctone is used as an anti-dandruff agent. In another embodiment, piroctone is used as a preservative. Piroctone, which is comprised in the cosmetic composition of the invention, is preferably used as an antifungal agent.

Preferably, the dandruff is caused by dandruff-causing organisms, more preferably Malassezia species, even more preferably Malassezia furfur and/or Malassezia globosa, particularly preferably Malassezia furfur, also particularly preferably Malassezia globosa.

The present invention also relates to a method of treating hair and/or scalp, comprising:
a) applying a shampoo composition and/or hair conditioner composition (preferably shampoo composition) as described herein onto wet hair and/or scalp and then
b) removing the shampoo composition and/or hair conditioner composition (preferably shampoo composition) from the hair and/or scalp.

The invention also relates to the use of piroctone as a replacement of piroctone olamine in a cosmetic composition. In preferred embodiments, the cosmetic composition comprises a fragrance. In a preferred embodiment, the fragrance comprises an aldehyde or ketone functional group. Preferred fragrances are described further above.

For example, the cosmetic composition of the invention may be selected from the group consisting of shampoo, hair conditioner, hair tonic, cream rinse, body wash, bubble bath, bath oil, facial cleanser, cleansing mask, cleansing milk, micellar water, make-up remover, cleansing wipes, perfume, soaps, shaving soaps, shaving foams, cleansing foams, face mask, face cream, hand cream and body lotion. In a particularly preferred embodiment, the cosmetic composition is an anti-dandruff shampoo.

Piroctone olamine is also known as piroctone ethanolamine. The chemical name for piroctone olamine is the monoethanolamine salt of 1 hydroxy-4-methyl-6-(2,4,4-trimethylpentyl)-2(1H)-pyridone. Hence, piroctone olamine is the ethanolamine salt of the hydroxamic acid derivative piroctone.

The invention also relates to the use of piroctone as a replacement of zinc pyrithione in a cosmetic composition. In preferred embodiments, the cosmetic composition comprises a fragrance. In a preferred embodiment, the fragrance comprises an aldehyde or ketone functional group. Preferred fragrances are described further above.

For example, the cosmetic composition of the invention may be selected from the group consisting of shampoo, hair conditioner, hair tonic, cream rinse, body wash, bubble bath, bath oil, facial cleanser, cleansing mask, cleansing milk, micellar water, make-up remover, cleansing wipes, perfume, soaps, shaving soaps, shaving foams, cleansing foams, face mask, face cream, hand cream and body lotion. In a particularly preferred embodiment, the cosmetic composition is an anti-dandruff shampoo.

If not stated otherwise, all percentages are by weight (w/w) of the total composition. If not stated otherwise, all ratios are weight ratios. "wt.%" means percentage by weight. "QS" or "QSP" means sufficient quantity for 100% or for 100g. "Molecular weight" or "M.Wt." or "MW" and grammatical equivalents mean the number average molecular weight, if not stated otherwise.

The invention is further illustrated by the following examples, without being limited thereby.

### Examples

### Example 1

**Bar shampoos**

| **Example** | **1a** | **1b** | **1c** | **1d** |
|---|---|---|---|---|
| Ingredient | wt.-% | wt.-% | wt.-% | wt.-% |
| Piroctone | 0.1 | 0.3 | 0.5 | 1.0 |
| Fragrance | 0.2 | 0.3 | 0.4 | 0.5 |
| Sodium Methyl Cocoyl Taurate | 6 | 6 | 6 | 6 |
| Sodium Cocoyl Isethionate | 60.5 | 60.5 | 60.5 | 60.5 |
| Cocamidopropyl Betaine | 3 | 3 | 3 | 3 |
| PEG-180 | 25 | 25 | 25 | 25 |
| Quaternium-98 (Genadvance Repair) | 4 | 4 | 4 | 4 |
| Preservative | As needed | | | |

### Example 2

**Cosmetic compositions (ingredients are given in wt%)**

| **Example** | **2a** | **2b** | **2c** | **2d** | **2e** |
|---|---|---|---|---|---|
| Composition | Liquid soap | Body wash | Wet wipe lotion | Shampoo | Shampoo |
| Piroctone | 0.5 | 0.5 | 0.5 | 0.5 | 0.5 |
| Fragrance | 0.3 | 0.3 | 0.3 | 0.3 | 0.3 |
| SLES | 10 | - | - | - | 9 |
| Cocamidopropyl betaine | 2 | 4.5 | - | - | 2 |
| Cocamide MEA | - | - | - | - | 1 |
| Sodium Cocoyl Glutamate | - | 2 | 1 | 2 | - |
| Sodium Cocoyl Glycinate | - | 2 | 1 | 2 | - |
| Sodium Cocoyl Isethionate | - | 2 | - | - | - |
| Cocoyl Methyl Glucamide | - | 3 | 2 | - | - |
| EGDS | - | 0.5 | - | - | 0.7 |
| PEG-7 Glyceryl Cocoate | - | - | 1 | - | - |
| Cocoyl Glucoside | - | - | - | 5 | - |
| Lauryl Glucoside | - | - | - | 5 | - |
| Acrylates Copolymer | - | - | - | 2 | - |
| Glycerin | - | - | - | 1 | 2 |
| Panthenol | - | - | - | 0.2 | 0.2 |
| Dimethicone | - | - | - | - | - |
| Polyquaternium 7 (PQ-7) | - | - | - | - | 0.6 |
| Guar Hydroxypropyltrimonium Chloride | - | - | - | - | 0.3 |
| Hydrolyzed Protein | - | - | - | - | 0.1 |
| N-fatty acyl-N-methyl cyclic glucamide | 1.5 | 2 | 1.0 | 1.5 | 2 |
| NaCl | 1.5 | 1.0 | 0.5 | 0.3 | 1.5 |
| Water | QSP | QSP | QSP | QSP | QSP |
| Total | 100 | 100 | 100 | 100 | 100 |

### Example 3

**Cosmetic compositions (ingredients are given in wt%)**

| **Example** | **3a** | **3b** | **3c** | **3d** |
|---|---|---|---|---|
| Composition | Body Lotion | Intensive hand and body lotion | Anti-ageing night cream | Baby cream |
| Piroctone | 0.2 | 0.2 | 0.2 | 0.1 |
| Fragrance | 0.3 | 0.3 | 0.2 | 0.1 |
| Ethyhexyl Stearate | 7 | - | - | - |
| Decyl Oleate | 5 | - | - | - |
| Plantasens^{®} Natural Emulsifier HE 20 (Clariant), which is Cetearyl Glucoside (and) Sorbitan Olivate | 3 | - | - | - |
| Dimethicone | 2 | - | - | - |
| Glycerin | 3 | 3 | 7 | - |
| Xanthan Gum | 0.2 | - | - | - |
| Aristoflex^{®} AVC from Clariant (Ammonium Acryloyldimethyltaurate / VP Copolymer) | 0.5 | 1 | - | - |
| Polyglyceryl-2-Sesquiisostearate | - | 0.5 | - | 2 |
| Trilaureth-4 Phosphate | - | 2 | - | - |
| Mineral Oil (Paraffinum Liquidum) | - | 5 | - | - |
| Plantasens^{®} Refined Organic Babassu Butter from Clariant (Orbignya Oleifera Seed Oil) | - | 2 | - | - |
| Squalane | - | 2 | 12 | - |
| Macadamia Ternifolia Seed Oil (and) Crambe Abyssinica Seed Oil (and) Orbignya Oleifera Seed Oil | - | 2 | - | - |
| Silcare^{®} Silicone SEA from Clariant (Trideceth-9 PG-Amodimethicone and Trideceth-12) | - | 0.5 | - | - |
| Cetearyl Alcohol | - | 2 | - | - |
| Isopropyl Palmitate | - | 4 | - | - |
| Dow Corning^{®} 345 (Cyclopentasiloxane and Cyclohexasiloxane) | - | - | 10 | - |
| Paraffin Oil, low viscosity | - | - | 4 | 10 |
| Petrolatum | - | - | 4 | 15 |
| Cetyl Alcohol | - | - | 3 | - |
| Cutina^{®} GMS (Glyceryl Stearate) | - | - | 2.5 | - |
| PEG-40 Stearate | - | - | 3 | - |
| Cera Alba Wax | - | - | 2 | - |
| Mangifera Indica (Mango) Seed Butter | - | - | 2 | - |
| Abyssinian Oil (and) Phytosterols (and) Olea Europea (Olive) Oil Unsaponifiables | - | - | 0.5 | - |
| Sorbitan Tristearate | - | - | 0.3 | - |
| Ubiquinone | - | - | 0.05 | - |
| Aristoflex^{®} Velvet from Clariant (Polyacrylate Crosspolymer-11 ) | - | - | 0.3 | - |
| Hostacerin^{®} SFO from Clariant (Sunflower Seed Oil Sorbitol Esters) | - | - | - | 3.5 |
| Paracera^{®} M (Cera Microcristallina) | - | - | - | 2 |
| Beeswax | - | - | - | 1 |
| Magnesium Stearate | - | - | - | 1 |
| Talc | - | - | - | 10 |
| Zinc oxide | - | - | - | 10 |
| Allantoin (Clariant) | - | - | - | 0.3 |
| Extrapon Hamamelis | - | - | - | 2 |
| D-Panthenol | - | - | - | 2 |
| N-fatty acyl-N-methyl cyclic glucamide | 1.5 | 2 | 1.0 | 1.5 |
| Water | QSP | QSP | QSP | QSP |
| Total | 100 | 100 | 100 | 100 |

### Example 4

**Cosmetic compositions (ingredients are given in wt%)**

| **Example** | **4a** | **4b** | **4c** | **4d** | **4e** | **4f** |
|---|---|---|---|---|---|---|
| Composition | Soap formulation | Soap bar | Body wash | Shampoo | Wash cream | Body wash |
| Piroctone | 0.5 | 1.0 | 0.5 | 0.5 | 0.5 | 0.5 |
| Fragrance | 0.4 | 1.0 | 0.3 | 0.3 | 0.2 | 0.2 |
| Sodium Lauryl Sulfate | - | - | 10 | - | - | - |
| Ammonium Lauryl Sulfate | - | - | - | 12 | - | - |
| Sodium Lauryl Ether Sulfate (SLES) | 15 | - | 2 | - | - | - |
| Cocamidopropyl betaine | 7 | - | 2 | 3 | - | 2 |
| Cocamide MEA | - | - | 1 | - | - | 2 |
| Lauric Acid | 0.5 | 2 | - | - | - | 10 |
| Myristic Acid | 1.5 | 2 | - | - | - | 10 |
| Stearic Acid | 0.5 | 2 | - | - | 10 | - |
| Palmitic Acid | - | 2 | - | - | - | 10 |
| Potassium Tallowate | - | 2 | - | - | - | - |
| Sodium Palm Kernelate | - | 20 | - | - | - | - |
| Sodium Cocoate | - | 60 | - | - | - | - |
| Lauryl Glucoside | - | - | - | 4 | - | - |
| Sodium Cocoyl Isethionate | - | - | - | - | 14 | - |
| Glycerin | - | 5 | - | - | - | 5 |
| Cetearyl Alcohol | 1.5 | - | - | - | 2 | - |
| Dimethicone | - | - | - | - | 0.1 | - |
| Polyquaternium 7 (PQ-7) | - | - | - | 0.2 | - | - |
| Guar Hydroxypropyltrimonium Chloride | - | - | 0.3 | - | - | - |
| Hydrolyzed Protein | - | - | - | 0.1 | - | - |
| N-fatty acyl-N-methyl cyclic glucamide | 1.5 | 2 | 1.0 | 1.5 | 1 | 0.8 |
| NaCl | 1.5 | - | 0.5 | 0.3 | - | 2.5 |
| Water | QSP | QSP | QSP | QSP | QSP | QSP |
| Total | 100 | 100 | 100 | 100 | 100 | 100 |

### Example 5

**Hair conditioner compositions**

| **Example** | **5a** | **5b** | **5c** | **5d** | **5e** | **5f** |
|---|---|---|---|---|---|---|
| Ingredient | wt.-% | wt.-% | wt.-% | wt.-% | wt.-% | wt.-% |
| Piroctone | 0.4 | 0.5 | 0.3 | 0.4 | 0.5 | 0.6 |
| Fragrance | 0.3 | 0.4 | 0.2 | 0.3 | 0.4 | 0.2 |
| Cetearyl Alcohol | 4.0 | 4.0 | 4.0 | 4.0 | 4.0 | 4.0 |
| CTAC | 2.0 | - | - | 1.0 | 1.0 | - |
| BTAC | - | 2.0 | - | 1.0 | - | 1.0 |
| BTMS | - | - | 2.0 | - | 1.0 | 1.0 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

### Example 6

**Hair conditioner compositions**

| **Example** | **6a** | **6b** | **6c** | **6d** | **6e** | **6f** |
|---|---|---|---|---|---|---|
| Ingredient | wt.-% | wt.-% | wt.-% | wt.-% | wt.-% | wt.-% |
| Piroctone | 0.4 | 0.5 | 0.3 | 0.4 | 0.5 | 0.6 |
| Fragrance | 0.2 | 0.3 | 0.4 | 0.2 | 0.3 | 0.4 |
| Cetearyl Alcohol | 4.0 | 4.0 | 4.0 | 4.0 | 4.0 | 4.0 |
| CTAC | 0.5 | 0.5 | 0.5 | - | - | - |
| BTAC | - | - | - | 0.5 | 0.5 | 0.5 |
| Genadvance^{®} Life | 1.5 | - | - | 1.5 | - | - |
| Genadvance^{®} Repair | - | 1.5 | - | - | 1.5 | - |
| Genadvance^{®} Hydra | - | - | 1.5 | - | - | 1.5 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

| | | | | | | |
|---|---|---|---|---|---|---|
| Genadvance^{®} Life: Clariant, INCI = Polyquaternium-116 und Butylene Glycol Genadvance^{®} Repair: Clariant, INCI = Quaternium-98 Genadvance^{®} Hydra: Clariant, INCI = Lauryl/Myristyl Polyricinoleate & Glycerin | | | | | | |

### Example 7

**Hair conditioner compositions**

| **Example** | **7a** | **7b** | **7c** | **7d** | **7e** | **7f** |
|---|---|---|---|---|---|---|
| Ingredient | wt.-% | wt.-% | wt.-% | wt.-% | wt.-% | wt.-% |
| Piroctone | 0.4 | 0.5 | 0.3 | 0.4 | 0.5 | 0.6 |
| Fragrance | 0.4 | 0.2 | 0.3 | 0.4 | 0.2 | 0.3 |
| Cetearyl Alcohol | 4.0 | 4.0 | 4.0 | 4.0 | 4.0 | 4.0 |
| CTAC | 0.2 | 0.2 | 0.2 | - | - | - |
| BTAC | - | - | - | 0.2 | 0.2 | 0.2 |
| Genadvance^{®} Life | 1.8 | - | - | 1.8 | - | - |
| Genadvance^{®} Repair | - | 1.8 | - | - | 1.8 | - |
| Genadvance^{®} Hydra | - | - | 1.8 | - | - | 1.8 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

| | | | | | | |
|---|---|---|---|---|---|---|
| Genadvance^{®} Life: Clariant, INCI = Polyquaternium-116 und Butylene Glycol Genadvance^{®} Repair: Clariant, INCI = Quaternium-98 Genadvance^{®} Hydra: Clariant, INCI = Lauryl/Myristyl Polyricinoleate & Glycerin | | | | | | |

### Example 8

**Shampoo compositions**

| **Example** | **8a** | **8b** | **8c** | **8d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.2 | 0.3 | 0.4 | 0.5 |
| sodium laureth sulfate | 8 | 8 | 8 | 8 |
| sodium lauryl sulfate | 8 | 8 | 8 | 8 |
| cocamide MIPA | 4 | 4 | 4 | 4 |
| glycerin | 3.5 | 3.5 | 3.5 | 3.5 |
| glycol distearate | 3 | 3 | 3 | 3 |
| sodium chloride | 2.5 | 2.5 | 2.5 | 2.5 |
| dimethicone | 2 | 2 | 2 | 2 |
| PPG-5-ceteth-20 | 1.5 | 1.5 | 1.5 | 1.5 |
| Hair conditioning agent | 1 | 1 | 1 | 1 |
| carbomer | 0.4 | 0.4 | 0.4 | 0.4 |
| niacinamide | 0.2 | 0.2 | 0.2 | 0.2 |
| pyridoxine HCl | 0.2 | 0.2 | 0.2 | 0.2 |
| guar hydroxypropyltrimonium chloride | 0.2 | 0.2 | 0.2 | 0.2 |
| hydrolyzed soy protein | 0.2 | 0.2 | 0.2 | 0.2 |
| methyl cocoate | 0.15 | 0.15 | 0.15 | 0.15 |
| sodium cocoate | 0.15 | 0.15 | 0.15 | 0.15 |
| citric acid | As needed, pH 4.5 - 6.5 | | | |
| sodium hydroxide | As needed, pH 4.5 - 6.5 | | | |
| Colorant | As needed | | | |

### Example 9

**Shampoo compositions**

| **Example** | **9a** | **9b** | **9c** | **9d** |
|---|---|---|---|---|
| Ingredient | wt% | wt % | wt% | wt% |
| Aqua | To 100 | To 100 | To 100 | To 100 |
| Piroctone | 0.1 | 0.3 | 0.7 | 1.0 |
| Fragrance | 0.2 | 0.3 | 0.4 | 0.5 |
| sodium laureth sulfate | 14 | 14 | 14 | 14 |
| coco-betaine | 4 | 4 | 4 | 4 |
| glycerin | 3 | 3 | 3 | 3 |
| glycol distearate | 2.5 | 2.5 | 2.5 | 2.5 |
| dimethicone | 2 | 2 | 2 | 2 |
| Hair conditioning agent | 1.5 | 1.5 | 1.5 | 1.5 |
| niacinamide | 0.5 | 0.5 | 0.5 | 0.5 |
| coconut oil | 0.5 | 0.5 | 0.5 | 0.5 |
| sodium chloride | 0.5 | 0.5 | 0.5 | 0.5 |
| olive fruit oil | 0.4 | 0.4 | 0.4 | 0.4 |
| PPG-5-ceteth-20 | 0.3 | 0.3 | 0.3 | 0.3 |
| trideceth-6 | 0.25 | 0.25 | 0.25 | 0.25 |
| polyquaternium-6 | 0.25 | 0.25 | 0.25 | 0.25 |
| amodimethicone | 0.2 | 0.2 | 0.2 | 0.2 |
| carbomer | 0.2 | 0.2 | 0.2 | 0.2 |
| cetrimonium chloride | 0.15 | 0.15 | 0.15 | 0.15 |
| citric acid | As needed, pH 4.5 - 6.5 | | | |
| sodium hydroxide | As needed, pH 4.5 - 6.5 | | | |
| Colorant | As needed | | | |

### Example 10

**Shampoo compositions**

| **Example** | **10a** | **10b** | **10c** | **10d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.3 | 0.2 | 0.5 | 0.4 |
| ammonium lauryl sulfate | 16 | 16 | 16 | 16 |
| cocoamidopropyl betaine | 5 | 5 | 5 | 5 |
| sodium chloride | 2.5 | 2.5 | 2.5 | 2.5 |
| niacinamide | 2 | 2 | 2 | 2 |
| Hair conditioning agent | 1 | 1 | 1 | 1 |
| hydroxypropyl guar hydroxypropyltrimonium chloride | 0.8 | 0.8 | 0.8 | 0.8 |
| propylene glycol | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 11

**Shampoo compositions**

| **Example** | **11a** | **11b** | **11c** | **11d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.3 | 0.2 | 0.5 | 0.4 |
| sodium laureth sulfate | 16 | 16 | 16 | 16 |
| glycol distearate | 4 | 4 | 4 | 4 |
| coco-betaine | 2.5 | 2.5 | 2.5 | 2.5 |
| glycerin | 2 | 2 | 2 | 2 |
| sodium chloride | 1.5 | 1.5 | 1.5 | 1.5 |
| dimethicone | 1.5 | 1.5 | 1.5 | 1.5 |
| Hair conditioning agent | 1.5 | 1.5 | 1.5 | 1.5 |
| niacinamide | 1 | 1 | 1 | 1 |
| guar hydroxypropyltrimonium chloride | 0.8 | 0.8 | 0.8 | 0.8 |
| cocamide MIPA | 0.5 | 0.5 | 0.5 | 0.5 |
| PPG-5-ceteth-20 | 0.2 | 0.2 | 0.2 | 0.2 |
| fumaric acid | 0.15 | 0.15 | 0.15 | 0.15 |
| carbomer | 0.15 | 0.15 | 0.15 | 0.15 |
| pyridoxine HCI | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 12

**Shampoo compositions**

| **Example** | **12a** | **12b** | **12c** | **12d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.3 | 0.3 | 0.3 | 0.3 |
| sodium laureth sulfate | 16 | 16 | 16 | 16 |
| coco-betaine | 4 | 4 | 4 | 4 |
| niacinamide | 2 | 2 | 2 | 2 |
| Ribes nigrum oil | 1.5 | 1.5 | 1.5 | 1.5 |
| cocamide MIPA | 1.5 | 1.5 | 1.5 | 1.5 |
| sodium chloride | 1.5 | 1.5 | 1.5 | 1.5 |
| Hair conditioning agent | 1.2 | 1.2 | 1.2 | 1.2 |
| sodium cocoate | 0.8 | 0.8 | 0.8 | 0.8 |
| cocamide MIPA | 0.5 | 0.5 | 0.5 | 0.5 |
| Olea europaea oil (olive) fruit oil | 0.3 | 0.3 | 0.3 | 0.3 |
| PEG-55 propylene glycol oleate | 0.3 | 0.3 | 0.3 | 0.3 |
| PEG-60 hydrogenated castor oil | 0.3 | 0.3 | 0.3 | 0.3 |
| polyquaternium-7 | 0.2 | 0.2 | 0.2 | 0.2 |
| amodimethicone | 0.15 | 0.15 | 0.15 | 0.15 |
| propylene glycol | 0.1 | 0.1 | 0.1 | 0.1 |
| Butyrospermum parkii butter | 0.1 | 0.1 | 0.1 | 0.1 |
| laureth-5 carboxylic acid | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 13

**Shampoo compositions**

| **Example** | **13a** | **13b** | **13c** | **13d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.2 | 0.2 | 0.2 | 0.2 |
| sodium laureth sulfate | 10 | 10 | 10 | 10 |
| disodium cocamphodiacetate | 5 | 5 | 5 | 5 |
| glycerin | 3 | 3 | 3 | 3 |
| glycol distearate | 2 | 2 | 2 | 2 |
| sodium chloride | 1.5 | 1.5 | 1.5 | 1.5 |
| Hair conditioning agent | 1.5 | 1.5 | 1.5 | 1.5 |
| cocamide MEA | 1 | 1 | 1 | 1 |
| polyquaternium-10 | 1 | 1 | 1 | 1 |
| carbomer | 0.3 | 0.3 | 0.3 | 0.3 |
| citric acid | As needed, pH 4.5 - 6.5 | | | |
| sodium hydroxide | As needed, pH 4.5 - 6.5 | | | |
| Colorant | As needed | | | |

### Example 14

**Shampoo compositions**

| **Example** | **14a** | **14b** | **14c** | **14d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.3 | 0.4 | 0.3 | 0.4 |
| sodium laureth sulfate | 12 | 12 | 12 | 12 |
| coco-betaine | 3 | 3 | 3 | 3 |
| cocamide MIPA | 2 | 2 | 2 | 2 |
| sodium chloride | 1.8 | 1.8 | 1.8 | 1.8 |
| Olea europaea (olive) fruit oil | 0.5 | 0.5 | 0.5 | 0.5 |
| Hair conditioning agent | 0.5 | 0.5 | 0.5 | 0.5 |
| PPG-5-ceteth-20 | 0.4 | 0.4 | 0.4 | 0.4 |
| PEG-55 propylene glycol oleate | 0.4 | 0.4 | 0.4 | 0.4 |
| PEG-60 hydrogenated castor oil | 0.25 | 0.25 | 0.25 | 0.25 |
| polyquaternium-10 | 0.25 | 0.25 | 0.25 | 0.25 |
| amodimethicone | 0.15 | 0.15 | 0.15 | 0.15 |
| propylene glycol | 0.15 | 0.15 | 0.15 | 0.15 |
| laureth-5 | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 15

**Shampoo compositions**

| **Example** | **15a** | **15b** | **15c** | **15d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.3 | 0.4 | 0.3 | 0.4 |
| ammonium lauryl sulfate | 15 | 15 | 15 | 15 |
| cocamidopropyl betaine | 3 | 3 | 3 | 3 |
| sodium chloride | 2 | 2 | 2 | 2 |
| propylene glycol | 1.8 | 1.8 | 1.8 | 1.8 |
| hydroxypropyl guar hydroxypropyltrimonium chloride | 1.5 | 1.5 | 1.5 | 1.5 |
| Hair conditioning agent | 1 | 1 | 1 | 1 |
| citric acid | As needed, pH 4.5 - 6.5 | | | |
| sodium hydroxide | As needed, pH 4.5 - 6.5 | | | |
| Colorant | As needed | | | |

### Example 16

**Shampoo compositions**

| **Example** | **16a** | **16b** | **16c** | **16d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.2 | 0.3 | 0.4 | 0.5 |
| sodium cocoyl isethionate | 4 | 4 | 4 | 4 |
| sodium lauryl sulfoacetate | 3 | 3 | 3 | 3 |
| disodium laureth sulfosuccinate | 2.5 | 2.5 | 2.5 | 2.5 |
| cocamidopropyl betaine | 2.5 | 2.5 | 2.5 | 2.5 |
| sodium lauroyl sarcosinate | 2.5 | 2.5 | 2.5 | 2.5 |
| glycol distearate | 2 | 2 | 2 | 2 |
| glycereth-26 | 1.8 | 1.8 | 1.8 | 1.8 |
| decyl glucoside | 1.5 | 1.5 | 1.5 | 1.5 |
| hydrogenated coconut acid | 1.4 | 1.4 | 1.4 | 1.4 |
| PPG-5-ceteth-20 | 1.3 | 1.3 | 1.3 | 1.3 |
| divinyldimethicone/dimethicone copolymer | 1.2 | 1.2 | 1.2 | 1.2 |
| sodium chloride | 1.1 | 1.1 | 1.1 | 1.1 |
| amodimethicone | 1 | 1 | 1 | 1 |
| Hair conditioning agent | 1 | 1 | 1 | 1 |
| polyquaternium-7 | 1 | 1 | 1 | 1 |
| polyquaternium-10 | 0.7 | 0.7 | 0.7 | 0.7 |
| sodium isethionate | 0.5 | 0.5 | 0.5 | 0.5 |
| PEG-55 propylene glycol oleate | 0.4 | 0.4 | 0.4 | 0.4 |
| propylene glycol | 0.4 | 0.4 | 0.4 | 0.4 |
| carbomer | 0.4 | 0.4 | 0.4 | 0.4 |
| C11-15 pareth-7 | 0.3 | 0.3 | 0.3 | 0.3 |
| glycerin | 0.3 | 0.3 | 0.3 | 0.3 |
| trideceth-12 | 0.25 | 0.25 | 0.25 | 0.25 |
| laureth-9 | 0.2 | 0.2 | 0.2 | 0.2 |
| hydroxypropyltrimonium hydrolyzed wheat protein | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 17

**Shampoo compositions**

| **Example** | **17a** | **17b** | **17c** | **17d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.2 | 0.3 | 0.4 | 0.5 |
| sodium methyl cocoyl taurate | 5 | 5 | 5 | 5 |
| laureth-5 carboxylic acid | 4 | 4 | 4 | 4 |
| sodium chloride | 2 | 2 | 2 | 2 |
| cocamidopropyl betaine | 2 | 2 | 2 | 2 |
| glycerin | 1.8 | 1.8 | 1.8 | 1.8 |
| glycol distearate | 1.5 | 1.5 | 1.5 | 1.5 |
| Hair conditioning agent | 1.5 | 1.5 | 1.5 | 1.5 |
| polyquaternium-10 | 1 | 1 | 1 | 1 |
| PPG-5 ceteth-20 | 0.5 | 0.5 | 0.5 | 0.5 |
| sodium lauroyl glutamate | 0.4 | 0.4 | 0.4 | 0.4 |
| propylene glycol | 0.2 | 0.2 | 0.2 | 0.2 |
| PEG-55 propylene glycol oleate | 0.15 | 0.15 | 0.15 | 0.15 |
| Argania spinosa kernel oil | 0.1 | 0.1 | 0.1 | 0.1 |
| citric acid, lactic acid | As needed, pH 4.5 - 6.5 | | | |
| sodium hydroxide | As needed, pH 4.5 - 6.5 | | | |
| Colorant | As needed | | | |

### Example 18

**Shampoo compositions**

| **Example** | **18a** | **18b** | **18c** | **18d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.3 | 0.3 | 0.4 | 0.4 |
| sodium laureth sulfate | 10 | 10 | 10 | 10 |
| disodium cocoamphodiacetate | 4 | 4 | 4 | 4 |
| glycol distearate | 2 | 2 | 2 | 2 |
| sodium chloride | 1.5 | 1.5 | 1.5 | 1.5 |
| Hair conditioning agent | 1.3 | 1.3 | 1.3 | 1.3 |
| carbomer | 0.4 | 0.4 | 0.4 | 0.4 |
| diaminopyrimidine oxide | 0.3 | 0.3 | 0.3 | 0.3 |
| disodium ricinoleamido MEA sulfosuccinate | 0.3 | 0.3 | 0.3 | 0.3 |
| propylene glycol | 0.2 | 0.2 | 0.2 | 0.2 |
| panthenol | 0.1 | 0.1 | 0.1 | 0.1 |
| polyquaternium-10 | 0.1 | 0.1 | 0.1 | 0.1 |
| citric acid | As needed, pH 4.5 - 6.5 | | | |
| sodium hydroxide, ammonium hydroxide | As needed, pH 4.5 - 6.5 | | | |
| Colorant | As needed | | | |

### Example 19

**Shampoo compositions**

| **Example** | **19a** | **19b** | **19c** | **19d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.3 | 0.3 | 0.4 | 0.4 |
| sodium cocoyl isethionate | 4.5 | 4.5 | 4.5 | 4.5 |
| sodium lauryl sulfoacetate | 3 | 3 | 3 | 3 |
| disodium laureth sulfosuccinate | 2.5 | 2.5 | 2.5 | 2.5 |
| cocamidopropyl betaine | 2.5 | 2.5 | 2.5 | 2.5 |
| sodium lauroyl sarcosinate | 2.3 | 2.3 | 2.3 | 2.3 |
| glycol distearate | 2 | 2 | 2 | 2 |
| glycereth-26 | 1.8 | 1.8 | 1.8 | 1.8 |
| decyl glucoside | 1.7 | 1.7 | 1.7 | 1.7 |
| hydrogenated coconut acid | 1.5 | 1.5 | 1.5 | 1.5 |
| PPG-5-ceteth-20 | 1.5 | 1.5 | 1.5 | 1.5 |
| PEG-55 propylene glycol oleate | 1.5 | 1.5 | 1.5 | 1.5 |
| propylene glycol | 1.5 | 1.5 | 1.5 | 1.5 |
| sodium chloride | 1.5 | 1.5 | 1.5 | 1.5 |
| divinyldimethicone/dimethicone copolymer | 1 | 1 | 1 | 1 |
| polyquaternium-7 | 1 | 1 | 1 | 1 |
| amodimethicone | 1 | 1 | 1 | 1 |
| Hair conditioning agent | 1 | 1 | 1 | 1 |
| polyquaternium-10 | 0.8 | 0.8 | 0.8 | 0.8 |
| sodium isethionate | 0.6 | 0.6 | 0.6 | 0.6 |
| carbomer | 0.6 | 0.6 | 0.6 | 0.6 |
| C11-15 pareth-7 | 0.4 | 0.4 | 0.4 | 0.4 |
| glycerin | 0.3 | 0.3 | 0.3 | 0.3 |
| laureth-9 | 0.2 | 0.2 | 0.2 | 0.2 |
| citric acid | As needed, pH 4.5 - 6.5 | | | |
| sodium hydroxide | As needed, pH 4.5 - 6.5 | | | |
| Colorant | As needed | | | |

### Example 20

**Shampoo compositions**

| **Example** | **20a** | **20b** | **20c** | **20d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.2 | 0.5 | 0.4 | 0.3 |
| cocamidopropyl betaine | 5 | 5 | 5 | 5 |
| sodium lauryl sulfoacetate | 4 | 4 | 4 | 4 |
| disodium laureth sulfosuccinate | 3.5 | 3.5 | 3.5 | 3.5 |
| sodium lauroyl sarcosinate | 3 | 3 | 3 | 3 |
| glycol distearate | 2 | 2 | 2 | 2 |
| sodium chloride | 1.7 | 1.7 | 1.7 | 1.7 |
| decyl glucoside | 1.5 | 1.5 | 1.5 | 1.5 |
| Hair conditioning agent | 1.5 | 1.5 | 1.5 | 1.5 |
| polyquaternium-10 | 1 | 1 | 1 | 1 |
| PPG-5-ceteth-20 | 1 | 1 | 1 | 1 |
| coco-betaine | 0.8 | 0.8 | 0.8 | 0.8 |
| PEG-55 propylene glycol oleate | 0.8 | 0.8 | 0.8 | 0.8 |
| propylene glycol | 0.5 | 0.5 | 0.5 | 0.5 |
| carbomer | 0.4 | 0.4 | 0.4 | 0.4 |
| citric acid | As needed, pH 4.5 - 6.5 | | | |
| sodium hydroxide | As needed, pH 4.5 - 6.5 | | | |
| Colorant | As needed | | | |

### Example 21

**Shampoo compositions**

| **Example** | **21a** | **21b** | **21c** | **21d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| Fragrance | 0.2 | 0.5 | 0.4 | 0.3 |
| sodium laureth sulfate | 15 | 15 | 15 | 15 |
| coco-betaine | 3 | 3 | 3 | 3 |
| glycol distearate | 2.5 | 2.5 | 2.5 | 2.5 |
| sodium chloride | 2 | 2 | 2 | 2 |
| PPG-5-ceteth-20 | 1.8 | 1.8 | 1.8 | 1.8 |
| Hair conditioning agent | 1.5 | 1.5 | 1.5 | 1.5 |
| octyldodecanol | 1 | 1 | 1 | 1 |
| carbomer | 1 | 1 | 1 | 1 |
| guar hydroxypropyltrimonium chloride | 1 | 1 | 1 | 1 |
| sodium hyaluronate | 0.4 | 0.4 | 0.4 | 0.4 |
| citric acid | As needed, pH 4.5 - 6.5 | | | |
| sodium hydroxide | As needed, pH 4.5 - 6.5 | | | |
| Colorant | As needed | | | |

## Claims

1. Cosmetic composition comprising piroctone and a fragrance.

2. Cosmetic composition according to claim 1, wherein the cosmetic composition comprises from 0.05 to 5 wt.-%, preferably from 0.1 to 2 wt.-%, more preferably from 0.1 to 1.0 wt.-%, particularly preferably from 0.1 to 0.5 wt.-% of piroctone, based on the total weight of the cosmetic composition.

3. Cosmetic composition according to claim 1 or 2, wherein the cosmetic composition comprises from 0.1 to 5 wt.-%, preferably from 0.1 to 2 wt.-%, more preferably from 0.1 to 1.0 wt.-%, particularly preferably from 0.1 to 0.5 wt.-% of the fragrance, based on the total weight of the cosmetic composition.

4. Cosmetic composition according to any of claims 1 to 3, wherein the fragrance comprises an aldehyde or ketone functional group.

5. Cosmetic composition according to any of claims 1 to 4, wherein the cosmetic composition is free of piroctone olamine.

6. Cosmetic composition according to any of claims 1 to 5, wherein the cosmetic composition is selected from the group consisting of shampoo, hair conditioner, hair tonic, cream rinse, body wash, bubble bath, bath oil, facial cleanser, cleansing mask, cleansing milk, micellar water, make-up remover, cleansing wipes, perfume, soaps, shaving soaps, shaving foams, cleansing foams, face mask, face cream, hand cream and body lotion.

7. Cosmetic composition according to any of claims 1 to 6, wherein the cosmetic composition is an anti-dandruff shampoo.

8. Use of piroctone as a replacement of piroctone olamine in a cosmetic composition.

9. Use according to claim 8, wherein the cosmetic composition comprises a fragrance.

10. Use according to claim 9, wherein the fragrance comprises an aldehyde or ketone functional group.

11. Use according to any of claims 8 to 10, wherein the cosmetic composition is selected from the group consisting of shampoo, hair conditioner, hair tonic, cream rinse, body wash, bubble bath, bath oil, facial cleanser, cleansing mask, cleansing milk, micellar water, make-up remover, cleansing wipes, perfume, soaps, shaving soaps, shaving foams, cleansing foams, face mask, face cream, hand cream and body lotion.

12. Use according to any of claims 8 to 11, wherein the cosmetic composition is an anti-dandruff shampoo.
